# EUROPÄISCHE PATENTSCHRIFT

(11) **EP 3 175 801 B1**
(45) Veröffentlichungstag und Bekanntmachung des Hinweises auf die Patenterteilung: **29.12.2021**
(21) Anmeldenummer: 16002430.3
(22) Anmeldetag: 16.11.2016
(51) Int. Cl.: A61B 17/29, F16D 3/18, F16H 1/00

(54) **MEDIZINISCHES INSTRUMENT**
MEDICAL INSTRUMENT
INSTRUMENT MÉDICAL

(30) Priorität: 01.12.2015 DE 102015015664
(43) Veröffentlichungstag der Anmeldung: 07.06.2017
(73) Patentinhaber: Karl Storz SE & Co. KG, 78532 Tuttlingen (DE)
(72) Erfinder: Thouément, Yann, F-78960 Les Essarts-le-Roi (FR); Besse, Régis, F-78610 Le Perray en Yvelines (FR); Jochen, Stefan, 78532 Tuttlingen (DE); Kärcher, Daniel, 78532 Tuttlingen (DE)
(74) Vertreter: Hofmeister, Frank

(56) Entgegenhaltungen:
- EP-A1- 0 630 614
- EP-B1- 2 036 505
- DE-A1- 10 036 108
- GB-A- 1 034 305
- US-A- 2 388 456
- US-A- 2 430 129
- US-A1- 2009 088 770

## Beschreibung

Die Erfindung betrifft ein medizinisches Instrument mit einem hohlen Schaft, an dessen proximalem Ende eine Handhabe angeordnet ist und an dessen distalem Ende ein Werkzeug so angeordnet ist, dass ein das Werkzeug tragender distaler Endbereich des Schaftes als gegenüber der Längsachse des Schaftes abwinkelbare Werkzeugspitze ausgebildet ist sowie das Werkzeug um die Längsachse des Schaftes bzw. um die Längsachse der Werkzeugspitze drehbar ist, wobei das Verdrehen des Werkzeugs um die Längsachse des Schaftes über eine in dem hohlen Schaft drehbar gelagerte Betätigungsstange erfolgt, die proximalseitig mit der Handhabe in Wirkverbindung steht und wobei die Betätigungsstange zweiteilig ausgebildet aus einem in der abwinkelbaren Werkzeugspitze gelagerten distalen Teilbereich sowie einem im proximalen Teil des Schaftes gelagerten Teilbereich besteht und wobei die beiden einander zugewandten Stirnseiten der Teilbereiche der Betätigungsstange im Übergang zur abwinkelbaren Werkzeugspitze über stirnseitige Verzahnungen miteinander in Eingriff stehen.

Medizinische Instrumente für die endoskopische Chirurgie weisen in der Regel einem hohlen Schaft auf, an dessen proximalem Ende eine Handhabe und an dessen distalem Ende ein Werkzeug angeordnet ist. Das als Greif- ,Halte- und/oder Schneidinstrument ausgebildete Werkzeug ist über die Handhabe betätigbar. Um bei den häufig beengten Arbeitsverhältnissen mit dem Werkzeug einen möglichst großen Wirkungsbereich abdecken zu können, sind viele endoskopische Instrumente so ausgebildet, dass das Werkzeug gegenüber der Längsachse des Schaftes abwinkelbar ausgebildet ist sowie das Werkzeug um die Längsachse des Schaftes drehbar ist.

Aus der US 2009/088770A1 ist ein gattungsgemäßer abgewinkelter chirurgischer Antrieb bekannt, bei dem die Werkzeugspitze und der Schaft über eine stirnseitige Verzahnung miteinander in Eingriff stehen. Die beiderseitigen Zähne sind dabei als mit abgerundeten Köpfen versehene Noppen ausgebildet. Die abgerundeten Noppen ermöglichen zwar ein verkantungsfreies Ineinandergreifen der beiderseitigen Zähne, jedoch liegen die Zähne zur Kraftübertragung nur punktuell aneinander an.

Weiterhin ist aus der DE 100 36 108 A1 ein chirurgisches Instrument bekannt, dessen Werkzeugspitze gegenüber dem Schaft über eine beiderseitige Verzahnung verschwenkbar ist, wobei die Verzahnung aus radial nach außen abstehenden Stiften an der Werkzeugspitze und Radialstiften am Schaft besteht, die miteinander kämmen. Diese Stiftverzahnung weist einerseits ein ungünstige Knickbelastung der einzelnen Stifte bei der kämmenden Verzahnung auf und andererseits ist diese Art der Verzahnung nur mit einem relativ großen Spiel der Stifte zueinander realisierbar.

Die US 2 430 129 A offenbart eine als Kugelverzahnung ausgebildete Stirnverzahnung, bei der die Zähne eines Zahnrads in der Draufsicht oval ausgebildet sind und die Zähne der korrespondierenden Verzahnung als klassische Stirnverzahnung ohne sich nach innen verjüngende Zahnflanken ausgebildet sind.

Ein weiteres medizinisches Instrument ist beispielsweise aus der EP 2 036 505 B1 bekannt. Um auch bei jedem Grad der Abwinklung der Werkzeugspitze gegenüber dem Schaft des Instruments einen stets ausreichenden und verkantungsfreien Eingriff der stirnseitigen Verzahnungen der Teilbereiche der Bestätigungsstange zu gewährleisten, ist bei diesem bekannten medizinischen Instrument der proximalseitige Teilbereich der Betätigungsstange über ein Federelement in Richtung auf den in der Werkzeugspitze gelagerten distalseitigen Teilbereich der Betätigungsstange vorgespannt.

Dieser Ausgleichsmechanismus gewährleistet zwar einen guten Eingriff der beiden stirnseitigen Verzahnungen, jedoch ist der konstruktive Aufwand sehr groß und steht der insbesondere bei endoskopischen Instrumenten erforderlichen kompakten Bauweise entgegen. Darüber hinaus ist die Verwendung von Federmechanismen aus reinigungstechnischer Sicht nachteilig, da diese häufig schlecht zugängliche Hinterschneidungen aufweisen.

Davon ausgehend liegt der Erfindung die **Aufgabe** zugrunde, ein medizinisches Instrument der eingangs genannten Art zu schaffen, das bei kompakter Bauweise eine gleichmäßige Maulteilrotation unabhängig von der Abwinklung der Werkzeugspitze und mit bestmöglicher und verkantungsfreier Kraftübertragung gewährleistet.

Die Lösung dieser Aufgabenstellung ist erfindungsgemäß dadurch gekennzeichnet, dass die Zahnflanken der einzelnen Zähne der beiden stirnseitigen Verzahnungen sich radial nach außen verjüngend ausgebildet sind und, dass die Zahnflanken der einzelnen Zähne der beiden stirnseitigen Verzahnungen zusätzlich sich radial nach innen verjüngend ausgebildet sind und wobei die beidseitigen Zahnflanken der einzelnen Zähne der beiden stirnseitigen Verzahnungen aus ebenen Teilflächen und gewölbten Teilflächen gebildet sind.

Durch die Ausbildung der Zahnflanken der einzelnen Zähne der beiden stirnseitigen Verzahnungen als sich nach radial außen und zusätzlich radial nach innen verjüngend, wird ein Verkanten der beiderseitigen Zähne der stirnseitigen Verzahnungen auch bei einem Verschwenken der Stirnverzahnungen relativ zueinander ausgeschlossen. Diese jederzeitige Verkantungsfreiheit macht es erstmalig möglich, auf den aus dem Stand der Technik bekannten Axialausgleich, beispielsweise in der Form einer Federvorspannung, zu verzichten.

Durch die zusätzliche Verjüngung der Zahnflanken auch in die nach innen gerichtete radiale Richtung wird die Bewegungsfreiheit der einzelnen Zähne der beiden stirnseitigen Verzahnungen relativ zueinander weiter erhöht.

Die beidseitigen Zahnflanken der einzelnen Zähne der beiden stirnseitigen Verzahnungen sind aus ebenen Teilflächen und gewölbten Teilflächen gebildet, um bei jeder Winkellage der stirnseitigen Verzahnungen relativ zueinander stets eine bestmögliche und verkantungsfreie Kraftübertragung von Zahn zu Zahn der beiden stirnseitigen Verzahnungen zu gewährleisten.

Weiterhin wird mit einer bevorzugten Ausführungsform der Erfindung vorgeschlagen, dass die Zahnköpfe der einzelnen Zähne der beiden stirnseitigen Verzahnungen abgerundet ausgebildet sind. Diese Abrundung der die Oberseite der einzelnen Zähne bildenden Zahnköpfe erleichtert weiterhin das verkantungsfreie Verschwenken der stirnseitigen Verzahnungen relativ zueinander.

Schließlich wird mit einer weiteren bevorzugten Ausführungsform der Erfindung vorgeschlagen, dass die beidseitig radial außen liegenden Teilflächen der Zahnflanken der einzelnen Zähne der beiden stirnseitigen Verzahnungen gewölbt ausgebildet sind. Die Ausbildung der radial außenliegenden Teilflächen der beidseitigen Zahnflanken als gewölbte Teilflächen erlaubt ein verkantungsfreies An- und Ablaufen der beiderseitigen Zahnräder aneinander insbesondere in den Positionen, in denen die stirnseitigen Verzahnungen relativ zueinander verschwenkt angeordnet sind.

Weitere Merkmale und Vorteile ergeben sich anhand der zugehörigen Zeichnungen, in denen ein Ausführungsbeispiel eines erfindungsgemäßen medizinischen Instruments nur beispielhaft dargestellt ist, ohne die Erfindung auf dieses Ausführungsbeispiel zu beschränken. In den Zeichnungen zeigt:
- Fig. 1: eine perspektivische Seitenansicht eines erfindungsgemäßen medizinischen Instruments;
- Fig. 2: eine teilweise transparente Seitenansicht des Details II gemäß Fig. 1, eine abgewinkelte Position darstellend;
- Fig. 3: einen ausschnittweisen Schnitt entlang der Linie III-III gemäß Fig. 1;
- Fig. 4: eine perspektivische Ansicht einer stirnseitigen Verzahnung gemäß Fig. 2;
- Fig. 5: eine perspektivische Ansicht der beiden stirnseitigen Verzahnung gemäß Fig. 2 in einer um 45° gegeneinander abgewinkelten Position und
- Fig. 6: eine perspektivische Ansicht der beiden stirnseitigen Verzahnung gemäß Fig. 2 in einer um 90° gegeneinander abgewinkelten Position.

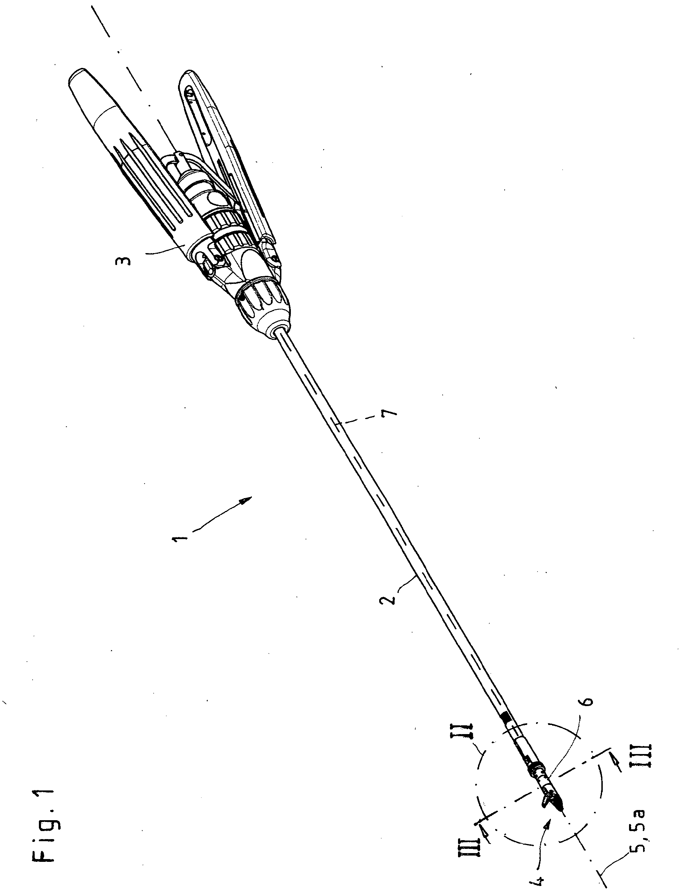

Die Abbildung Fig. 1 zeigt ein medizinisches Instrument 1 mit einem hohlen Schaft 2, an dessen proximalem Ende eine Handhabe 3 angeordnet ist und an dessen distalem Ende ein Werkzeug 4 angeordnet ist.

Um dem Werkzeug 4 möglichst viele Freiheitsgrade zur Bewegung relativ zum Schaft 2 einzuräumen, ist ein das Werkzeug 4 tragender distaler Endbereich des Schaftes 2 als gegenüber der Längsachse 5 des Schaftes 2 abwinkelbare Werkzeugspitze 6 ausgebildet ist.

Weiterhin ist das Werkzeug 4 um die Längsachse 5 des Schaftes 2 drehbar, wobei das Verdrehen des Werkzeugs 4 um die Längsachse 5 des Schaftes 2 bzw. bei abgewinkelter Werkzeugspitze 6 um die Längsachse 5a der Werkzeugspitze 6 über eine in dem hohlen Schaft 2 drehbar gelagerte Betätigungsstange 7 erfolgt, die proximalseitig mit der Handhabe 3 in Wirkverbindung steht, wobei die Betätigungsstange 7 zweiteilig ausgebildet aus einem in der abwinkelbaren Werkzeugspitze 6 gelagerten distalen Teilbereich 8 sowie einem im proximalen Teil des Schaftes 2 gelagerten Teilbereich 9 besteht.

Zur Ausbildung der Betätigungsstange 7 kann sowohl eine massive Stange als auch ein Hohlrohr verwendet werden.

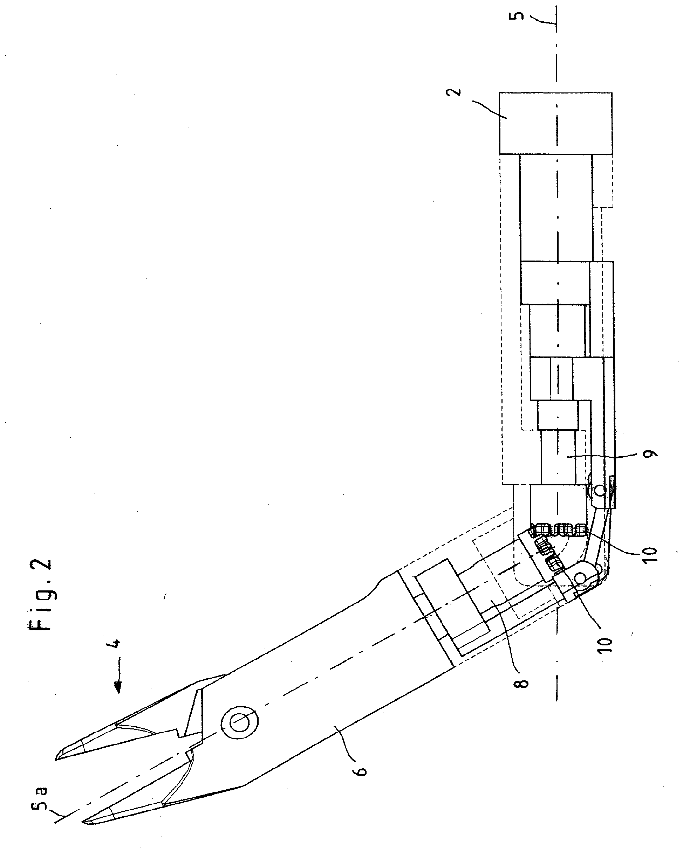

Die beiden einander zugewandten Stirnseiten der Teilbereiche 8 und 9 der Betätigungsstange 7 stehen im Übergang zur abwinkelbaren Werkzeugspitze 6 über stirnseitige Verzahnungen 10 miteinander in Eingriff, wie dies der schematischen Schnittdarstellung gemäß Fig. 2 zu entnehmen ist. Diese beiden stirnseitigen Verzahnungen 10 der beiden Teilbereiche 8 und 9 der Betätigungsstange 7 ermöglichen es, dass das Werkzeug 4 auch in einer Position, in der die beiden Teilbereiche 8 und 9 der Betätigungsstange 7 relativ zueinander abgewinkelt angeordnet sind, um die Längsachse 5 des Schaftes 2 bzw. um die Längsachse 5a der Werkzeugspitze 6 drehbar ist. Die stirnseitigen Verzahnungen 10 übertragen die Rotation des proximalen Teilbereichs 9 der Betätigungsstange 7 um die Längsachse 5 des Schaftes 2 auf den distalen Teilbereich 8 der Betätigungsstange 7.

Die Abbildung Fig. 2 zeigt die Anordnung der beiden stirnseitigen Verzahnungen 10 innerhalb der Schaftes 2 anhand einer teilweise transparenten schematischen Seitenansicht im Bereich des Übergangs vom proximalen Schaft 2 zur abgewinkelten distalen Werkzeugspitze 6.

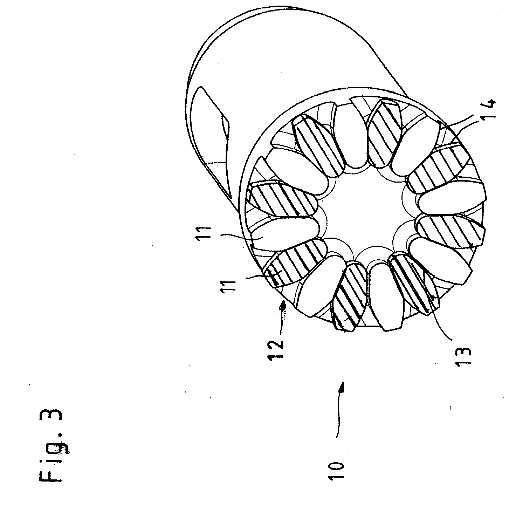

Die Abbildung Fig. 3 zeigt die beiden stirnseitigen Verzahnungen 10 in der nicht abgewinkelten Position der Teilbereiche 8 und 9 der Betätigungsstange 7, in der alle Zähne 11 der jeweiligen stirnseitigen Verzahnungen 10 der beiden Teilbereiche 8 und 9 der Betätigungsstange 7 in Eingriff miteinander stehen.

Ein Hauptproblem bei der Ausbildung der beiden stirnseitigen Verzahnungen 10 der relativ zueinander verschwenkbaren Teilbereiche 8 und 9 der Betätigungsstange 7 besteht darin, dass sich die Zähne 11 der stirnseitigen Verzahnungen 10 beim Verschwenken nicht gegenseitig verkanten und blockieren.

Wie aus Fig. 3 ersichtlich, sind die Zahnflanken 12 der einzelnen Zähne 11 der beiden stirnseitigen Verzahnungen 10 sich radial nach außen verjüngend ausgebildet. Durch die Ausbildung der Zahnflanken 12 der einzelnen Zähne 11 der beiden stirnseitigen Verzahnungen 10 als sich, in Bezug auf die jeweilige Verzahnung 10, nach radial außen verjüngend, wird ein Verkanten der beiderseitigen Zähne 11 der stirnseitigen Verzahnungen 10 auch bei einem Verschwenken der stirnseitigen Verzahnungen 10 relativ zueinander ausgeschlossen. Diese jederzeitige Verkantungsfreiheit macht es möglich, dass auf einen Axialausgleich, beispielsweise in der Form einer Federvorspannung, verzichtet werden kann. Aufgrund der sich radial nach außen verjüngenden Ausbildung der einzelnen Zähne 11 der beiden stirnseitigen Verzahnungen 10 verbleibt gerade im radial außen liegenden Bereich für die einzelnen Zähne 11 immer ausreichend seitlicher Spielraum zu den benachbarten Zähnen 11 der jeweils anderen stirnseitigen Verzahnung 10.

Wie aus den Abbildungen ersichtlich, sind bei den stirnseitigen Verzahnungen 10 zusätzlich auch die Zahnflanken 12 der einzelnen Zähne 11 der beiden stirnseitigen Verzahnungen 10 als sich, in Bezug auf die jeweilige Verzahnung 10, radial nach innen verjüngend ausgebildet, wodurch die Bewegungsfreiheit der einzelnen Zähne 11 der beiden stirnseitigen Verzahnungen 10 relativ zueinander weiter erhöht wird.

Wie weiterhin insbesondere aus der Abbildung Fig. 3 ersichtlich, weisen die beidseitigen Zahnflanken 12 der einzelnen Zähne 11 der beiden stirnseitigen Verzahnungen 10 ebene Teilflächen 13 und gewölbte Teilflächen 14 auf, wobei die beidseitig radial außen liegenden Teilflächen 14 der Zahnflanken 12 der einzelnen Zähne 11 der beiden stirnseitigen Verzahnungen 10 gewölbt ausgebildet sind.

Die Ausbildung der radial außenliegenden Teilflächen 14 der beidseitigen Zahnflanken 12 als gewölbte Teilflächen 14 erlaubt ein verkantungsfreies An- und Ablaufen der beiden stirnseitigen Verzahnungen 10 aneinander, insbesondere in den Positionen, in denen die stirnseitigen Verzahnungen 10 relativ zueinander verschwenkt angeordnet sind.

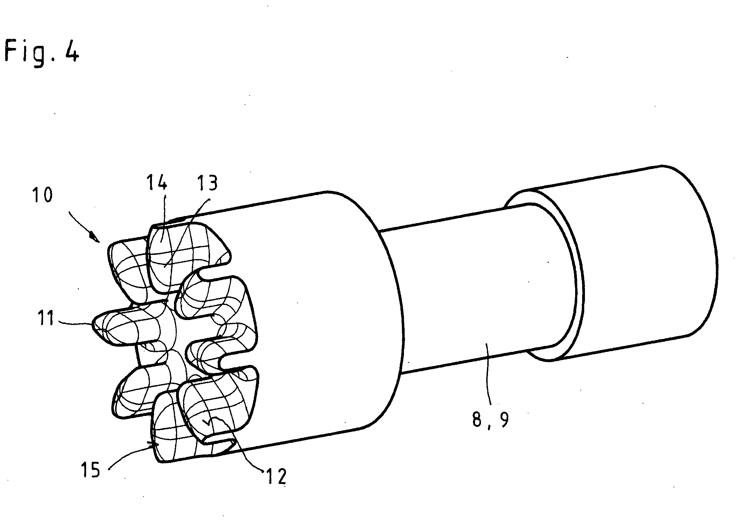

Der perspektivischen Ansicht gemäß Fig. 4 ist weiterhin zu entnehmen, dass die Zahnköpfe 15 der einzelnen Zähne 11 der beiden stirnseitigen Verzahnungen 10 abgerundet ausgebildet sind. Auch diese Abrundung der die Oberseite der einzelnen Zähne 11 bildenden Zahnköpfe15 erleichtert weiterhin das verkantungsfreie Verschwenken der stirnseitigen Verzahnungen 10 relativ zueinander.

Die wie zuvor beschrieben ausgebildeten stirnseitigen Verzahnungen 10 zeichnen sich dadurch aus, dass sie in allen Winkelstellungen der Teilbereiche 8 und 9 der Betätigungsstange 7 relativ zueinander ein verkantungsfreies Ablaufen der einzelnen Zähne 11 aufeinander gewährleisten und trotzdem auf einen Axialausgleich verzichtet werden kann.

Aufgrund der verschiedenen Winkellagen der stirnseitigen Verzahnungen 10 zueinander muss auf eine Evolventenverzahnung verzichtet werden. Durch die Ausbildung der sich radial nach außen verjüngenden Zahnflanken 12 der einzelnen Zähne 11 der beiden stirnseitigen Verzahnungen 10 sowie dem Aufbau der beidseitigen Zahnflanken 12 der einzelnen Zähne 11 aus ebenen Teilflächen 13 und gewölbten Teilflächen 14 ist die Geometrie der stirnseitigen Verzahnungen 10 so ausgelegt, dass in allen relativ zueinander verschwenkten Winkellagen die gewölbten Teilflächen 14 der einen stirnseitigen Verzahnung 10 entweder auf den ebenen Teilflächen 13 oder den gewölbten Teilflächen 14 der anderen stirnseitigen Verzahnung 10 verkantungsfrei ablaufen.

In der in Fig. 3 dargestellten nicht verschwenkten Ausrichtung der Teilbereiche 8 und 9 der Betätigungsstange 7 zueinander greifen die Zähne 11 ineinander, ohne dass ein Abrollen der Zahnflanken 12 erforderlich ist. In dieser Position liegen die ebenen Teilflächen 13 der Zahnflanken 12 aneinander an.

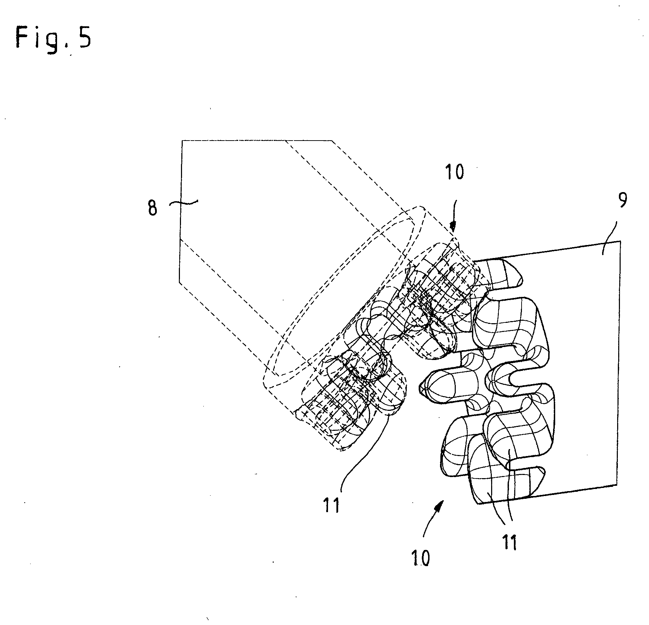

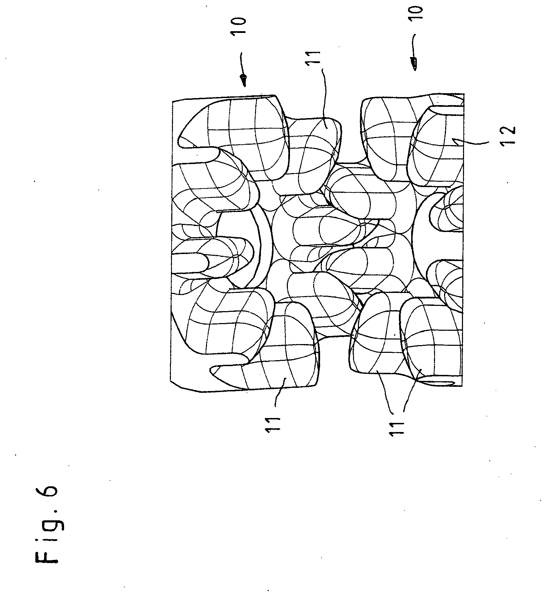

Die Abbildungen Fig. 5 und 6 zeigen die stirnseitigen Verzahnungen 10 in einer um 45° (Fig. 5) und einer um 90° (Fig. 6) relativ zueinander verschwenkten Position der stirnseitigen Verzahnungen 10 bzw. der Teilbereiche 8 und 9 der Betätigungsstange 7 zueinander. In diesen relativ zueinander verschwenkten Positionen laufen die Zähne 11 der stirnseitigen Verzahnungen 10 nur an den gewölbten Teilbereichen 14 der Zahnflanken 12 aneinander ab und übertragen so das Torsionsmoment vom proximalen Teilbereich 9 der Betätigungsstange 7 auf den distalen Teilbereich 8 der Betätigungsstange 7, wobei immer mindestens ein Zahn 11 der einen stirnseitigen Verzahnung 10 mit einem Zahn 11 der anderen stirnseitigen Verzahnung 10 in Eingriff steht.

### Bezugszeichenliste

- 1: medizinisches Instrument
- 2: Schaft
- 3: Handhabe
- 4: Werkzeug
- 5: Längsachse (Schaft)
- 5a: Längsache (Werkzeugspitze)
- 6: Werkzeugspitze
- 7: Betätigungsstange
- 8: Teilbereich (distal)
- 9: Teilbereich (proximal)
- 10: stirnseitige Verzahnung
- 11: Zahn
- 12: Zahnflanke
- 13: Teilfläche (eben)
- 14: Teilfläche (gewölbt)
- 15: Zahnkopf

## Patentansprüche

1. Medizinisches Instrument mit einem hohlen Schaft (2), an dessen proximalem Ende eine Handhabe (3) angeordnet ist und an dessen distalem Ende ein Werkzeug (4) so angeordnet ist, dass ein das Werkzeug (4) tragender distaler Endbereich des Schaftes (2) als gegenüber der Längsachse (5) des Schaftes (2) abwinkelbare Werkzeugspitze (6) ausgebildet ist sowie das Werkzeug (4) um die Längsachse (5) des Schaftes (2) bzw. um die Längsachse (5a) der Werkzeugspitze (6) drehbar ist, wobei das Verdrehen des Werkzeugs (4) um die Längsachse (5) des Schaftes (2) über eine in dem hohlen Schaft (2) drehbar gelagerte Betätigungsstange (7) erfolgt, die proximalseitig mit der Handhabe (3) in Wirkverbindung steht und wobei die Betätigungsstange (7) zweiteilig ausgebildet aus einem in der abwinkelbaren Werkzeugspitze (6) gelagerten distalen Teilbereich (8) sowie einem im proximalen Teil des Schaftes (2) gelagerten Teilbereich (9) besteht und wobei die beiden einander zugewandten Stirnseiten der Teilbereiche (8 und 9) der Betätigungsstange (7) im Übergang zur abwinkelbaren Werkzeugspitze (6) über stirnseitige Verzahnungen (10) miteinander in Eingriff stehen,
**dadurch gekennzeichnet,**
**dass** die Zahnflanken (12) der einzelnen Zähne (11) der beiden stirnseitigen Verzahnungen (10) sich radial nach außen verjüngend ausgebildet sind und, dass die Zahnflanken (12) der einzelnen Zähne (11) der beiden stirnseitigen Verzahnungen (10) zusätzlich sich radial nach innen verjüngend ausgebildet sind und wobei die beidseitigen Zahnflanken (12) der einzelnen Zähne (11) der beiden stirnseitigen Verzahnungen (10) aus ebenen Teilflächen (13) und gewölbten Teilflächen (14) gebildet sind.

2. Medizinisches Instrument nach Anspruch 1, **dadurch gekennzeichnet, dass** die Zahnköpfe (15) der einzelnen Zähne (11) der beiden stirnseitigen Verzahnungen (10) abgerundet ausgebildet sind.

3. Medizinisches Instrument nach Anspruch 1, **dadurch gekennzeichnet, dass** die beidseitig radial außen liegenden Teilflächen (14) der Zahnflanken (12) der einzelnen Zähne (11) der beiden stirnseitigen Verzahnungen (10) gewölbt ausgebildet sind.

## Claims

1. Medical instrument with a hollow shaft (2), at the proximal end of which a handle (3) is arranged, and at the distal end of which a tool (4) is arranged such that a distal end region of the shaft (2) carrying the tool (4) is designed as a tool tip (6) that is deflectable with respect to the longitudinal axis (5) of the shaft (2), and the tool (4) is rotatable about the longitudinal axis (5) of the shaft (2) and about the longitudinal axis (5a) of the tool tip (6), wherein the rotation of the tool (4) about the longitudinal axis (5) of the shaft (2) is effected via an actuation rod (7) which is mounted rotatably in the hollow shaft (2) and which is operatively connected at its proximal end to the handle (3), and wherein the actuation rod (7) is composed of two parts, namely a distal sub-region (8) mounted in the deflectable tool tip (6) and a sub-region (9) mounted in the proximal part of the shaft (2), and wherein the two mutually facing end faces of the sub-regions (8 and 9) of the actuation rod (7) are in engagement with each other at the transition to the deflectable tool tip (6) via end-face toothing arrangements (10), **characterized**
**in that** the tooth flanks (12) of the individual teeth (11) of the two end-face toothing arrangements (10) are designed tapering radially outwards, and in that the tooth flanks (12) of the individual teeth (11) of the two end-face toothing arrangements (10) are additionally designed tapering radially inwards, and wherein the tooth flanks (12) on both sides of the individual teeth (11) of the two end-face toothing arrangements (10) are composed of flat surface parts (13) and of curved surface parts (14).

2. Medical instrument according to Claim 1, **characterized in that** the tooth heads (15) of the individual teeth (11) of the two end-face toothing arrangements (10) are rounded.

3. Medical instrument according to Claim 1, **characterized in that** the radially outward surface parts (14) of the tooth flanks (12) on both sides of the individual teeth (11) of the two end-face toothing arrangements (10) are curved.

## Revendications

1. Instrument médical comprenant une tige creuse (2), à l'extrémité proximale de laquelle est disposée une poignée (3) et à l'extrémité distale de laquelle est disposé un outil (4) de telle sorte qu'une zone d'extrémité distale de la tige (2), qui porte l'outil (4), soit conçue comme une pointe d'outil (6) qui peut être inclinée par rapport à l'axe longitudinal (5) de la tige (2) et que l'outil (4) puisse tourner sur l'axe longitudinal (5) de la tige (2) ou sur l'axe longitudinal (5a) de la pointe d'outil (6), la rotation de l'outil (4) sur l'axe longitudinal (5) de la tige (2) s'effectuant par le biais d'une barre d'actionnement (7) qui est montée de manière rotative dans la tige creuse (2) et qui est reliée fonctionnellement du côté proximal à la poignée (3) et la barre d'actionnement (7) formée en deux parties comprenant une zone partielle distale (8) logée dans la pointe d'outil inclinable (6) et une zone partielle (9) montée dans la partie proximale de la tige (2) et les deux côtés frontaux, dirigés l'un vers l'autre, des zones partielles (8 et 9) de la barre d'actionnement (7) étant en engagement l'un avec l'autre par le biais d'une denture (10) au niveau de la transition vers la pointe d'outil inclinable (6),
caractérisé en que
les flancs (12) des dents individuelles (11) des deux dentures frontales (10) sont conçus de manière à se rétrécir radialement vers l'extérieur et,
les flancs (12) des dents individuelles (11) des deux dentures frontales (10) sont en outre conçus de manière à se rétrécir radialement vers l'intérieur et les flancs bilatéraux (12) des dents individuelles (11) des deux dentures frontales (10) étant formés de surfaces partielles planes (13) et de surfaces partielles bombées (14).

2. Instrument médical selon la revendication 1, **caractérisé en ce que** les pointes (15) des dents individuelles (11) des deux dentures frontales (10) sont conçues pour être arrondies.

3. Instrument médical selon la revendication 1, **caractérisé en ce que** les surfaces partielles (14), situées radialement vers l'extérieur des deux côtés, des flancs (12) des dents individuelles (11) des deux dentures frontales (10) sont conçues pour être bombées.
